(19) Europäisches Patentamt
European Patent Office
Office européen des brevets

(11) Veröffentlichungsnummer: **0 132 231**
**A2**

(12) # EUROPÄISCHE PATENTANMELDUNG

(21) Anmeldenummer: **84810347.9**

(22) Anmeldetag: **13.07.84**

(51) Int. Cl.⁴: **C 07 F 9/65**
**A 61 K 31/675**

(30) Priorität: **19.07.83 CH 3946/83**

(43) Veröffentlichungstag der Anmeldung: **23.01.85 Patentblatt 85/4**

(84) Benannte Vertragsstaaten: **AT BE CH DE FR GB IT LI LU NL SE**

(71) Anmelder: **CIBA-GEIGY AG**
**Postfach**
**CH-4002 Basel(CH)**

(72) Erfinder: **Storni, Angelo, Dr.**
**Im Feuerbusch 3**
**CH-4310 Rheinfelden(CH)**

(54) **Substituierte Thiazolidinylester von Mineralsäureamiden.**

(57) Die Erfindung betrifft neue Verbindungen der allgemeinen Formel

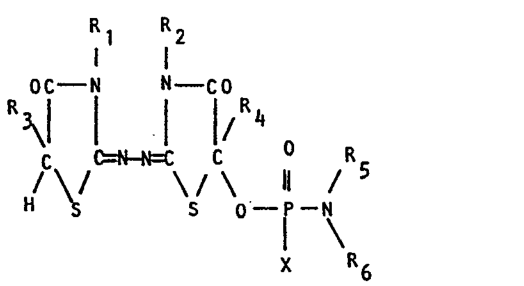

I,

in welcher eines der Symbole $R_1$ und $R_2$ einen in der 2,3-Stellung ungesättigten Alkylrest mit 3 oder 4 Kohlenstoffatomen und das andere einen solchen Rest oder Niederalkyl bedeuten, $R_3$ und $R_4$ unabhängig voneinander Wasserstoff oder Methyl darstellen, $R_5$ und $R_6$ unabhängig voneinander Wasserstoff, Niederalkyl, halogeniertes Niederalkyl oder Niederalkenyl oder zusammen gegebenenfalls durch Sauerstoff, Schwefel oder gegebenenfalls substituierten Stickstoff unterbrochenes Niederalkylen bedeuten, X $OR_7$ oder $NR_8R_9$ bedeutet, wobei $R_7$ für Wasserstoff oder Niederalkyl steht, und $R_8$ und $R_9$ unabhängig voneinander Wasserstoff, Niederalkyl, halogeniertes Niederalkyl oder Niederalkenyl oder zusammen gegebenenfalls durch Sauerstoff, Schwefel oder gegebenenfalls substituierten Stickstoff unterbrochenes Niederalkylen bedeuten, oder $R_6$ zusammen mit $R_7$ oder mit $R_8$ eine Niederalkylengruppe bedeutet, und Salze von Verbindungen der allgemeinen Formel I, in denen $R_7$ Wasserstoff bedeutet. Diese Verbindungen und ihre Salze zeigen Antitumor-Wirkungen.

EP 0 132 231 A2

Croydon Printing Company Ltd.

CIBA-GEIGY AG
Basel (Schweiz)

4-14507/+

## Substituierte Thiazolidinylester von Mineralsäureamiden

Gegenstand der Erfindung sind neue substituierte Thiazolidinylester von Mineralsäureamiden und Salze solcher Verbindungen mit wertvollen pharmakologischen Eigenschaften, Verfahren zur Herstellung dieser neuen Stoffe, sowie pharmazeutische Präparate, welche diese Stoffe enthalten, und die Verwendung dieser Stoffe und sie enthaltender Präparate.

Die erfindungsgemässen Verbindungen entsprechen der allgemeinen Formel

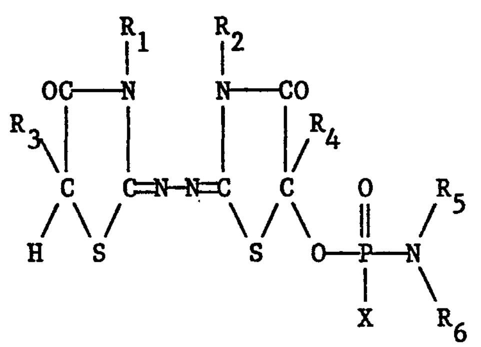

I,

in welcher eines der Symbole $R_1$ und $R_2$ einen in der 2,3-Stellung ungesättigten Alkylrest mit 3 oder 4 Kohlenstoffatomen und das andere einen solchen Rest oder Niederalkyl bedeuten, $R_3$ und $R_4$ unabhängig voneinander Wasserstoff oder Methyl darstellen, $R_5$ und $R_6$ unabhängig voneinander Wasserstoff, Niederalkyl, halogeniertes Niederalkyl oder Niederalkenyl oder zusammen gegebenenfalls durch Sauerstoff, Schwefel oder gegebenenfalls substituierten Stickstoff unterbrochenes Niederalkylen bedeuten, X $OR_7$ oder $NR_8R_9$ bedeutet, wobei $R_7$ für Wasserstoff oder Niederalkyl steht, und $R_8$ und $R_9$ unabhängig voneinander Wasserstoff, Niederalkyl, halogeniertes

Niederalkyl oder Niederalkenyl oder zusammen gegebenenfalls durch Sauerstoff, Schwefel oder gegebenenfalls substituierten Stickstoff unterbrochenes Niederalkylen bedeuten, oder $R_6$ zusammen mit $R_7$ oder mit $R_8$ eine Niederalkylengruppe Alk bedeutet.

Ebenfalls Gegenstand der Erfindung sind die Salze von Verbindungen der allgemeinen Formel I, in denen $R_7$ Wasserstoff bedeutet, mit Basen, insbesondere die pharmazeutisch annehmbaren Salze solcher Verbindungen.

In den Verbindungen der allgemeinen Formel I enthält ein in der 2,3-Stellung ungesättigter Alkylrest $R_1$ und/oder $R_2$ eine Doppel- oder Dreifachbindung und stellt z.B. entsprechendes Niederalkenyl, wie Allyl, 1-oder 2-Methylallyl, oder entsprechendes Niederalkinyl, z.B. 2-Propinyl, dar.

Niederalkyl enthält bis 7, vorzugsweise bis 4 Kohlenstoffatome und ist beispielsweise Pentyl, Isopentyl, Neopentyl, Hexyl, Heptyl, vorzugsweise Propyl, 2-Propyl, Butyl, Isobutyl, vor allem aber Ethyl und in erster Linie Methyl.

Halogeniertes Niederalkyl enthält bis 7, vorzugsweise bis 4 Kohlenstoffatome, ist in 1-, 2-, 3- oder auch höherer Stellung ein- oder mehrfach, insbesondere ein- oder zweifach, halogeniert, wie z.B. bromiert oder vorzugsweise chloriert, und ist z.B. Chlorpropyl, wie 1-, 2- oder 3-Chlorpropyl, oder insbesondere Chlorethyl, wie 1-oder 2-Chlorethyl, Dichlorethyl, wie 1,1-, 1,2-oder 2,2-Dichlorethyl, oder Chlormethyl.

Ein Niederalkenylrest enthält bis 7, vorzugsweise 3 bis 5 Kohlenstoffatome und die Doppelbindung insbesondere in einer höheren als der 1-Stellung und ist z.B. Allyl, 1- oder 2-Methylallyl, But-2-en-1-yl, Pent-2- oder -3-en-1-yl, aber auch 1- oder 2-Methylvinyl.

Gegebenenfalls durch Sauerstoff, Schwefel oder gegebenenfalls substituierten, wie Niederalkyl, z.B. Methyl, enthaltenden Stickstoff unterbrochenes Niederalkylen, das durch $R_5$ und $R_6$ bzw. $R_8$ und $R_9$ zusammen gebildet werden kann, enthält vorzugsweise 4 bis 5 Kettenatome und ist z.B. 1,4-Butylen, 1,5-Pentylen, 3-Oxa-1,5-pentylen, 3-Thia-1,5-pentylen, 3-Aza-1,5-pentylen oder 3-Methyl-3-aza-1,5-pentylen.

Ein Rest $R_6$, der mit $R_7$ zusammen eine Niederalkylengruppe Alk bildet, trennt die beiden Heteroatome vorzugsweise durch 2 bis 4 Kohlenstoffatome und stellt z.B. eine 1,4-Butylen-, eine 1,3-Propylen- und in erster Linie eine 1,2-Ethylengruppe dar, so dass $R_6$ und $R_7$ unter Einbeziehung der Atomgruppierung N-P-O z.B. substituiertes Perhydro-1,3,2-oxazaphosphepin oder -phosphorin und in erster Linie 1,3,2-Oxazaphospholidin bedeuten.

Ein Rest $R_6$ kann zusammen mit $R_8$ einen Niederalkylenrest Alk darstellen, der die beiden Stickstoffatome vorzugsweise durch 2 bis 4 Kohlenstoffatome trennt, z.B. eine 1,4-Butylen-, eine 1,3-Propylen- und in erster Linie eine 1,2-Ethylengruppe, so dass $R_6$ und $R_8$ unter Einbeziehung der Atomgruppierung N-P-N z.B. substituiertes Perhydro-1,3,2-diazaphosphepin oder -phosphorin und in erster Linie 1,3,2-Diazaphospholidin bedeuten.

Salze mit Basen von zur Salzbildung fähigen Verbindungen der allgemeinen Formel I, d.h. solchen, in denen X für Hydroxy steht, sind z.B. Metall-, wie Alkalimetall-, z.B. Natrium- oder Kaliumsalze, oder Erdalkalimetall-, wie Magnesium-oder Calciumsalze, ferner Ammoniumsalze und Salze mit primären, sekundären oder tertiären einsäurigen oder mehrsäurigen organischen Basen, wie z.B. mit Ethylamin, 2-Aminoethanol, Diethylamin, Iminodiethanol, Triethylamin, 2-(Diethylamino)-ethanol, Nitrilotriethanol oder Pyridin, bzw. 1,2-Ethandiamin. Bevorzugt sind die entsprechenden pharmazeutisch annehmbaren, nicht-toxischen Salze.

Das den Rest $R_4$ tragende Kohlenstoffatom und, sofern $R_3$ von Wasserstoff verschieden ist, das den Rest $R_3$ tragende Kohlenstoffatom in Verbindungen der Formel I sind asymmetrisch substituiert. Die Verbindungen der Formel I können demgemäss in Form von Isomerengemischen, z.B. Gemischen von Racematen (Diasteromerengemisch) oder in Form von reinen Isomeren, z.B. reinen Racematen bzw. optischen Antipoden vorliegen.

Die neuen Verbindungen der allgemeinen Formel I und die Salze solcher Verbindungen besitzen wertvolle pharmakologische Eigenschaften, insbesondere Tumor-hemmende Wirksamkeit. Diese kann im Tierversuch festgestellt werden, z.B. bei oraler oder parenteraler, wie intraperitonealer oder subcutaner Verabreichung von Dosen zwischen 10 und 250 mg/kg am Ehrlich-Karzinom der Maus (Transplantat: 1 x $10^6$ Zellen (Ascites) i.p. an weibliche Mäuse NMRI), am Walker-Karzinosarkom 256 der Ratte (Transplantat: 0,5 ml einer Suspension von soliden Tumoren in Hanks'-Lösung s.c. oder i.m. an männlichen Ratten (Wistar)), am transplantablen Mamma-Adenokarzinom R 3230 AC der Ratte (Transplantat: 0.5 ml einer Suspension von soliden Tumoren in Hanks'-Lösung s.c. oder i.m. an weiblichen Ratten (Fischer)), am subkutan transplantierten B16-Melanom der Maus (Transplantat: 0,2 ml einer Tumor-Suspension in Hanks'-Lösung s.c.) und am durch 7,12-Dimethylbenz[α]anthracen (DMBA) induzierten Mammakarzinom der Ratte (induziert durch p.o. Verabreichung von 15 mg DMBA in 1 ml Sesamöl an 50 Tage alte weibliche Ratten (Sprague Dawley), wobei multiple Tumoren nach 6 bis 8 Wochen festgestellt werden können).

So kann bei der Behandlung von weiblichen Sprague-Dawley-Ratten, die multiple hormonabhängige DMBA-induzierte Mammatumoren tragen, mit erfindungsgemässen Verbindungen, z.B. Phosphorsäure-dimethylamid-methylester-[3-methyl-2-[[5-methyl-3-(2-methylallyl)-4-oxo-2-thiazolidinyliden]-hydrazono]-4-oxo-5-thiazolidinyl]-ester (Beispiel 1) und Phosphorsäure-dimethylamid-[3-methyl-2-[[5-methyl-3-(2-methylallyl)-4-oxo-2-thiazolidinyliden]-hydrazono]-4-oxo-5-thiazolidinyl]-ester (Natriumsalz, Beispiel 4), eine erhebliche Hemmung des

Tumorwachstums und die Regression der Tumoren festgestellt werden [Applikation: 30 x 25 mg/kg oral bzw. 30 x 10 mg/kg subkutan; Methodik: K.H. Schmidt-Ruppin et al, Experientia 29, 823-825 (1973)]. Das Erscheinen neuer Tumoren wird sowohl bei oraler als auch bei subkutaner Verabreichung nachhaltig unterdrückt. Das Wachstum von subkutan transplantiertem B16-Melanom in der Maus wird durch 10 orale Verabreichungen (fünfmal pro Woche während 2 Wochen) von 250 mg/kg bzw. 125 mg/kg erfindungsgemässer Verbindungen sehr stark inhibiert, im Falle des Phosphorsäure-dimethylamid-methylester-[3-methyl-2-[[5-methyl-3-(2-methylallyl)-4-oxo-2-thiazolidinyliden]-hydrazono]-4-oxo-5-thiazolidinyl]-esters beispielsweise um 53% bzw. um 42%.

Im Vergleich zur starken Antitumor-Wirksamkeit sind die Toxizität und die Nebenwirkungen der erfindungsgemässen Verbindungen gering bis mässig. So beträgt die einmalige, maximal tolerierte Dosis in der Maus beispielsweise im Falle des Phosphorsäure-dimethylamid-methylester-[3-methyl-2-[[5-methyl-3-(2-methylallyl)-4-oxo-2-thiazolidinyliden]-hydrazono]-4-oxo-5-thiazolidinyl]-esters 1250-2500 mg/kg (p.o.) bzw. 500-1250 mg/kg (i.p.) und im Falle des Natriumsalzes des Phosphorsäure-dimethylamid-[3-methyl-2-[[5-methyl-3-(2-methylallyl)-4-oxo-2-thiazolidinyliden]-hydrazono]-4-oxo-5-thiazolidinyl]-esters mehr als 2500 mg/kg (p.o. und i.p.).

Die erfindungsgemässen Verbindungen der Formel I können demgemäss als solche oder insbesondere in Form von pharmazeutischen Präparaten zur Behandlung von hormonabhängigen und hormonunabhängigen neoplastischen Krankheiten bei Warmblütern, insbesondere zur Behandlung des Mammakarzinoms und des Prostatakarzinoms, durch enterale, insbesondere orale, oder parenterale Verabreichung von therapeutisch wirksamen Dosen, Verwendung finden.

Die Erfindung betrifft insbesondere Verbindungen der allgemeinen Formel I, in denen einer der Reste $R_1$ und $R_2$ in 2,3-Stellung ungesättigtes Alkyl mit 3 oder 4 Kohlenstoffatomen, z.B. entsprechendes Niederalkenyl oder entsprechendes Niederalkinyl, und der

andere ebenfalls eine dieser Gruppen oder Niederalkyl mit bis zu 4 Kohlenstoffatomen bedeuten, $R_3$ und $R_4$ unabhängig voneinander Wasserstoff oder Methyl darstellen, $R_5$ und $R_6$ unabhängig voneinander Wasserstoff, Niederalkyl, ein- oder zweifach halogeniertes Niederalkyl oder Niederalkenyl oder zusammen gegebenenfalls durch Sauerstoff, Schwefel oder gegebenenfalls niederalkylierten Stickstoff unterbrochenes Niederalkylen bedeuten, X $OR_7$ oder $NR_8R_9$ bedeutet, worin $R_7$ für Wasserstoff oder Niederalkyl steht, und $R_8$ und $R_9$ unabhängig voneinander Wasserstoff, Niederalkyl, ein- oder zweifach halogeniertes Niederalkyl oder Niederalkenyl oder zusammen gegebenenfalls durch Sauerstoff, Schwefel oder gegebenenfalls niederalkylierten Stickstoff unterbrochenes Niederalkylen bedeuten, oder $R_6$ zusammen mit $R_7$ oder mit $R_8$ Niederalkylen mit 2 bis 4 Kohlenstoffatomen bedeutet, und Salze von solchen Verbindungen der Formel I, worin $R_7$ Wasserstoff ist.

Die Erfindung betrifft vor allem Verbindungen der Formel I, worin einer der Reste $R_1$ und $R_2$ Allyl, 1-Methylallyl, 2-Methylallyl oder 2-Propinyl und der andere ebenfalls eine dieser Gruppen oder Methyl ist, $R_3$ und $R_4$ unabhängig voneinander für Wasserstoff oder Methyl stehen, $R_5$ und $R_6$ unabhängig voneinander Wasserstoff, Niederalkyl oder durch Chlor mono- oder disubstituiertes Niederalkyl oder zusammen gegebenenfalls durch Sauerstoff, Schwefel oder gegebenenfalls methylierten Stickstoff unterbrochenes Niederalkylen bedeuten, X $OR_7$ oder $NR_8R_9$ bedeutet, worin $R_7$ Wasserstoff oder Niederalkyl ist, und $R_8$ und $R_9$ unabhängig voneinander Wasserstoff, Niederalkyl oder durch Chlor mono- oder disubstituiertes Niederalkyl oder zusammen gegebenenfalls durch Sauerstoff, Schwefel oder gegebenenfalls methylierten Stickstoff unterbrochenes Niederalkylen bedeuten, oder $R_6$ zusammen mit $R_7$ oder mit $R_8$ Niederalkylen mit 2 bis 4 Kohlenstoffatomen bedeutet, und Salze von solchen Verbindungen der Formel I, worin $R_7$ Wasserstoff ist.

Vor allem betrifft die Erfindung Verbindungen der Formel I, in denen einer der Reste $R_1$ und $R_2$ Allyl oder 2-Methylallyl und der andere Methyl ist, $R_3$ Wasserstoff oder Methyl bedeutet, $R_4$ Wasserstoff

ist, $R_5$ und $R_6$ unabhängig voneinander Wasserstoff, Methyl oder 2-Chlorethyl bedeuten, X $OR_7$ oder $NR_8R_9$ ist, worin $R_7$, $R_8$ und $R_9$ Wasserstoff oder Methyl sind, oder $R_6$ zusammen mit $R_7$ Ethylen bedeutet, das zusammen mit der Atomgruppe O-P-N einen fünfgliedrigen Ring bildet, oder $R_6$ zusammen mit $R_8$ Ethylen bedeutet, das zusammen mit der Atomgruppe N-P-N einen fünfgliedrigen Ring bildet, und Salze von solchen Verbindungen der Formel I, in denen $R_7$ Wasserstoff ist.

Die Erfindung betrifft in erster Linie Verbindungen der Formel I, worin einer der Reste $R_1$ und $R_2$ Allyl oder 2-Methylallyl und der andere Methyl ist, $R_3$ für Wasserstoff oder Methyl steht, $R_4$ Wasserstoff ist, $R_5$ und $R_6$ Methyl sind, X $OR_7$ ist, worin $R_7$ Wasserstoff oder Methyl ist, oder $R_6$ zusammen mit $R_7$ Ethylen bedeutet, das zusammen mit der Atomgruppe -O-P-N- einen fünfgliedrigen Ring bildet, und Salze von solchen Verbindungen der Formel I, worin $R_7$ Wasserstoff ist.

Die Erfindung betrifft weiterhin die reinen optischen Antipoden von Verbindungen der Formel I sowie Mischungen dieser optischen Antipoden, z.B. Racemate oder Diastereomerengemische davon.

Vorrangig betrifft die Erfindung die in den Beispielen beschriebenen Verbindungen und Salze, vorzugsweise pharmazeutisch annehmbaren Salze, wie z.B. Alkalimetallsalze von entsprechenden salzbildenden Verbindungen, und ganz besonders den Phosphorsäure-dimethylamid-methylester-[3-methyl-2-[[5-methyl-3-(2-methylallyl)-4-oxo-2-thiazolidinyliden]-hydrazono]-4-oxo-5-thiazolidinyl]-ester, den Phosphorsäure-dimethylamid-[3-methyl-2-[[5-methyl-3-(2-methylallyl)-4-oxo-2-thiazolidinyliden]-hydrazono]-4-oxo-5-thiazolidinyl]-ester und dessen pharmazeutisch annehmbare Salze, wie z.B. die entsprechenden Alkalimetallsalze, z.B. das Natriumsalz, ferner das 3-Methyl-2-[3-methyl-2-[[5-methyl-3-(2-methylallyl)-4-oxo-2-thiazolidinyliden]-hydrazono]-4-oxo-5-thiazolidinoxy]-2-oxo-1,3,2-oxazaphospholidin und das 2-[3-Allyl-2-[[3-methyl-4-oxo-2-thiazolidinyliden]-hydrazono]-4-oxo-5-thiazolidinoxy]-3-methyl-2-oxo-1,3,2-oxazaphospholidin.

Die neuen Verbindungen der allgemeinen Formel I können nach an sich bekannten Verfahren hergestellt werden. So kann man sie z.B. herstellen, indem man

a) eine Verbindung der Formel

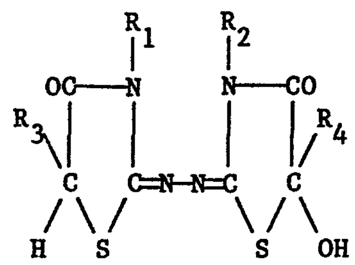

II,

mit einer den Phosphorsäurerest der Formel

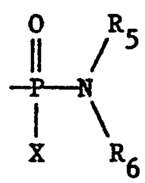

III

einführenden Verbindung umsetzt, oder

b) in einer Verbindung der Formel

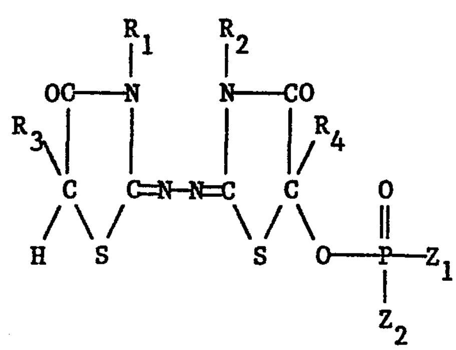

IV,

worin $Z_1$ einen durch die Gruppe $NR_5R_6$ ersetzbaren Rest oder $NR_5R_6$ und $Z_2$ einen durch die Gruppe X ersetzbaren Rest oder X bedeuten, mit der Massgabe, dass mindestens eine der Gruppen $Z_1$ und $Z_2$ von der Gruppe $NR_5R_6$ bzw. X verschieden ist, den Rest $Z_1$ durch die Gruppe $NR_5R_6$ und/oder den Rest $Z_2$ durch die Gruppe X ersetzt, und gewünschtenfalls eine erhältliche Verbindung der allgemeinen Formel I in eine andere Verbindung der allgemeinen Formel I überführt, und/oder

gewünschtenfalls ein verfahrensgemäss erhältliches Salz in die freie Verbindung oder in ein anderes Salz überführt und/oder eine verfahrensgemäss erhältliche Verbindung der Formel I, in der $R_7$ Wasserstoff bedeutet, in ein Salz derselben überführt, und/oder, wenn erwünscht, ein verfahrensgemäss erhältliches Isomerengemisch in die einzelnen Isomeren auftrennt.

Verfahren a) : Die Ausgangsstoffe der Formel II sind bekannt (z.B. Deutsche Offenlegungsschrift 2 405 395) oder sind analog zu den dort beschriebenen Verfahren herstellbar.

Einen Rest der Formel III einführende Verbindungen sind solche der allgemeinen Formel

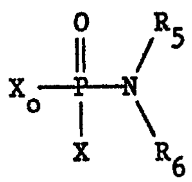

V,

in denen $X_o$ reaktionsfähiges funktionell abgewandeltes Hydroxy bedeutet. $X_o$ ist z.B. insbesondere mit einer anorganischen oder organischen Säure verestertes Hydroxy oder verethertes Hydroxy, wie mit einer Halogenwasserstoffsäure oder einer Aryl- oder Alkansulfonsäure, z.B. p-Toluolsulfonsäure, Methan- oder Ethansulfonsäure verestertes Hydroxy, oder z.B. durch einen aliphatischen, cycloaliphatischen, aromatischen oder araliphatischen Rest, wie einen entsprechenden, gegebenenfalls substituierten Kohlenwasserstoffrest verethertes Hydroxy, wie u.a. Aryloxy, wie Phenoxy oder p-Nitrophenoxy, oder Arylniederalkoxy, wie insbesondere Benzyloxy, ferner p-Nitrobenzyloxy, sowie Niederalkenyloxy, z.B. Allyloxy, ferner Niederalkoxy, z.B. Methoxy. Insbesondere bedeutet eine Gruppe $X_o$ z.B. Halogen, wie Brom und vor allem Chlor.

Ausgangsstoffe der Formel V sind bekannt oder können analog zu bekannten Verbindungen hergestellt werden.

Die Umsetzung von Verbindungen der Formel II mit solchen der Formel V wird vorzugsweise in einem inerten, insbesondere aprotischen organischen Lösungsmittel, wie z.B. Methylenchlorid, Acetonitril, Dimethylformamid oder Dimethylsulfoxid, und, wenn $X_o$ verestertes Hydroxy ist, vorzugsweise in Gegenwart eines säurebindenden Mittels, wie einer organischen Base, z.B. Triniederalkylamin, wie Ethyldiisopropylamin oder Triethylamin, ferner z.B. Pyridin oder Imidazol, oder Alkalimetallniederalkoxid, z.B. Natrium-methoxid oder -ethoxid, oder einer anorganischen Base, z.B. Natrium- oder Kaliumhydroxid, oder eines basischen Ionenaustauschers durchgeführt. Als Reaktionstemperatur wird z.B. eine solche zwischen etwa -10° und etwa 100°C, vorzugsweise Raumtemperatur oder schwach erhöhte oder erniedrigte Temperatur, z.B. bei etwa 0° bis etwa 40°C, gewählt, und die Reaktion kann, wenn notwendig, in einem geschlossenen Gefäss und/oder in einer Inertgas-, wie Stickstoffatmosphäre, durchgeführt werden.

Verfahren b): In Verbindungen der Formel IV bedeuten ein durch die Gruppe $NR_5R_6$ ersetzbarer Rest $Z_1$ Hydroxy oder reaktionsfähiges funktionell abgewandeltes Hydroxy und ein durch die Gruppe X ersetzbarer Rest $Z_2$ ein von Niederalkoxy verschiedenes reaktionsfähiges funktionell abgewandeltes Hydroxy. Eine derartige funktionell abgewandelte Hydroxygruppe $Z_1$ oder $Z_2$ hat z.B. die oben für $X_o$ gegebenen Bedeutungen, und ist insbesondere Halogen, wie Brom und vor allem Chlor.

Ausgangsverbindungen der Formel IV sind z.B. entsprechende Phosphorsäuretriester, Phosphorsäurediester, Phosphorsäuremonoester, Phosphorsäure-chlorid-diester, Phosphorsäure-amid-chlorid-ester oder Phosphorsäure-dichlorid-ester der Formel IV oder entsprechende -bromide oder -dibromide. Derartige Verbindungen der Formel IV sind neu, bis auf Phosphorsäuretriester, in denen $Z_1$ und $Z_2$ für Niederalkoxy stehen, Phosphorsäurediester, in denen $Z_1$ oder $Z_2$ für Niederalkoxy steht und Phosphorsäuremonoester der Formel IV, und bilden ebenfalls einen Gegenstand der vorliegenden Erfindung.

Umwandlung von $Z_2$ in Hydroxy: So können z.B. Verbindungen der Formel IV, in denen $Z_1$ eine Gruppe $NR_5R_6$ und $Z_2$ verestertes oder von Niederalkoxy verschiedenes verethertes Hydroxy bedeutet, in eine Verbindung der Formel I, worin X für Hydroxy steht, übergeführt werden, indem man z.B. eine veresterte Hydroxygruppen $Z_2$, wie Halogen, durch die Einwirkung von Wasser, gegebenenfalls in Form von Gemischen mit geeigneten organischen Lösungsmitteln, wie Dioxan oder Niederalkanolen, zu einer Hydroxygruppe hydrolysiert. Die gleichen Endstoffe kann man durch basische Hydrolyse sowohl aus den vorgenannten Verbindungen der Formel IV, als auch aus solchen mit Aryloxy- oder Aralkoxygruppen als $Z_2$ erhalten, z.B. durch Einwirkung von mindestens äquimolaren Mengen Wasser in Gegenwart von Basen, vorzugsweise in wasserhaltigen organischen Lösungsmitteln, wie entsprechenden Niederalkanolen oder Dioxan. Als Basen kann man hierbei sowohl organische, vorzugsweise tertiäre, wie die unter Verfahren a) genannten, oder anorganische Basen, wie Alkalimetall-, z.B. Natrium-oder Kaliumcarbonate oder hydroxide, verwenden, wobei ein Ueberschuss vermieden werden sollte. Man kann die Reaktionsprodukte entweder direkt als Salze oder, nach Behandeln mit einem sauren Reagens, als freie Säuren erhalten.

Verbindungen der Formel I, in denen X $OR_7$ und $R_7$ Wasserstoff bedeuten, lassen sich auch durch Behandeln einer Verbindung der Formel IV, worin $Z_2$ eine wie oben angegeben veretherte und von Niederalkoxy verschiedene Hydroxygruppe ist, mit einer starken anorganischen Base, wie einem Alkalimetall-, z.B. Natrium- oder Kaliumhydroxid, vorzugsweise in Gegenwart eines Alkohols, wie Niederalkanols, z.B. Ethanol, oder Ammoniumhydroxid, oder mit einem geeigneten Neutralsalz, insbesondere einem Alkali-oder Erdalkalimetallhalogenid oder -thiocyanat, wie Natriumiodid, Bariumiodid oder Natriumthiocyanat, erhalten, wobei sich diese Methode in erster Linie zur Spaltung von Niederalkenyloxy-, z.B. Allyloxy-, oder Arylniederalkoxy-, z.B. Benzyloxygruppen eignet.

Ferner lassen sich geeignet veretherte Hydroxygruppen $Z_2$, insbesondere araliphatisch veretherte Hydroxygruppen, wie gegebenenfalls substituiertes Benzyloxy, mittels Hydrogenolyse, wie durch Behandeln mit Wasserstoff in Gegenwart eines Edelmetallkatalysators, wie eines Platin- oder Palladiumkatalysators, spalten, wobei darauf geachtet werden muss, dass eine Niederalkenylgruppe $R_1$ bzw. $R_2$ nicht ebenfalls reduziert wird.

Umwandlung von $Z_2$ in Niederalkoxy oder $NR_8R_9$: Weiter kann man Verbindungen der Formel IV, worin $Z_2$ eine veresterte Hydroxygruppe darstellt, in Verbindungen der Formel I, worin X für $OR_7$, worin $R_7$ Niederalkyl bedeutet, oder für die Gruppe $NR_8R_9$ steht, überführen, indem eine entsprechende Verbindung der Formel IV, worin $Z_2$ z.B. Halogen bedeutet, z.B. mit einem Niederalkanol oder einer Verbindung der Formel $HNR_8R_9$ (VI) in Gegenwart einer Base unter im wesentlichen wasserfreien Reaktionsbedingungen oder mit einem Niederalkoxid eines Alkali- oder Erdalkalimetalls, wie einem Natrium- oder Kaliummeth--oxid, -ethoxid oder tert.-butoxid, umgesetzt wird.

Umwandlung von $Z_1$ in $NR_5R_6$: Ferner kann man Verbindungen der Formel IV, in denen $Z_2$ für X steht, und $Z_1$ für reaktionfähiges funktionell abgewandeltes Hydroxy, z.B. entsprechend verestertes Hydroxy, wie Halogen, z.B. Chlor steht, mit einer Verbindung der Formel $HNR_5R_6$ (VII), z.B. nach dem oben beschriebenen Verfahren, behandeln und in eine Verbindung der Formel I überführen. Ferner kann man ein Ausgangsmaterial der Formel IV, worin $Z_1$ für Hydroxy steht, z.B. durch Umsetzen mit einem geeigneten Reagens, wie mit N,N'-Dicyclohexylcarbodiimid oder Carbonyldiimidazol, und dann mit einem Amin der Formel VII in eine Verbindung der Formel I überführen.

Umwandlung von $Z_1$ und $Z_2$ in $NR_5R_6$ und X: Ausgangsverbindungen der Formel IV, in denen $Z_1$ und $Z_2$ wie oben beschriebenes reaktionsfähiges funktionell abgewandeltes Hydroxy oder $Z_2$ einen solchen Rest, der von Niederalkoxy verschieden ist, und $Z_1$ Hydroxy darstellen, lassen sich direkt in Verbindungen der Formel I überführen. Bei derartigen Ausgangsverbindungen der Formel IV kann es sich z.B. um

Phosphorsäure-dichlorid-ester oder -dibromid-ester der Formel IV handeln. So kann man sie mit einer Verbindung der Formel VI bzw. VII umsetzen und zu Verbindungen der Formel I gelangen, in denen X und $NR_5R_6$ die gleiche Bedeutung haben.

Ferner kann man solche Ausgangsverbindungen der Formel IV mit einer Verbindung der Formel

$$Y—Alk—NHR_5 \qquad VIII,$$

worin Y Hydroxy oder eine mit $R_9$ substituierte Aminogruppe bedeutet, und Alk Alkylen bedeutet, das Y vom Stickstoffatom durch 2 bis 4 Kohlenstoffatome trennt, zu einer Verbindung der Formel I umsetzen, in der $R_6$ mit X und der Atomgruppierung P-N einen fünf- bis siebengliedrigen Ring bildet. Ausgangsverbindungen der Formel VIII sind bekannt oder können auf bekannte Weise hergestellt werden.

Die Umsetzung von Verbindungen der Formel IV zu Verbindungen der Formel I wird vorzugsweise in einem inerten, insbesondere aprotischen organischen Lösungmittel, wie z.B. Methylenchlorid, Acetonitril, Dimethylformamid oder Dimethylsulfoxid, und, wenn $Z_1$ und/oder $Z_2$ verestertes Hydroxy sind, vorzugsweise in Gegenwart eines säurebindenden Mittels, wie einer organischen Base, z.B. Triniederalkylamin, wie Ethyldiisopropylamin oder Triethylamin, ferner z.B. Pyridin oder Imidazol, oder Alkalimetallniederalkoxid, z.B. Natrium-methoxid oder -ethoxid, oder einer anorganischen Base, z.B. Natrium- oder Kaliumhydroxid, oder eines basischen Ionenaustauschers durchgeführt. Als Reaktionstemperatur wird z.B. eine solche zwischen 0° und etwa 100°C, vorzugsweise Raumtemperatur oder schwach erhöhte oder erniedrigte Temperatur gewählt, und die Reaktion kann, wenn notwendig, in einem geschlossenen Gefäss, und/oder in einer Inertgas-, wie Stickstoffatmosphäre, durchgeführt werden.

Ausgangsstoffe der Formel IV können z.B. hergestellt werden, indem man eine Verbindung der Formel II mit einer Verbindung der Formel

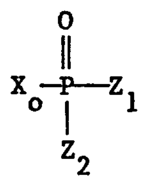

IX,

worin $X_o$ die unter Formel V angegebene Bedeutung hat, umsetzt. Als Ausgangsstoffe der Formel IX kommen deshalb z.B. halogenierte und/oder veresterte Phosphorsäuren oder Phosphorsäureamide in Betracht, wie Phosphorsäure-chlorid-ester, Phosphorsäure-chlorid-diester, Phosphorsäure-amid-dichloride oder auch entsprechende -bromide oder -dibromide.

Ausgangsstoffe der Formel IX sind bekannt oder können analog zu bekannten Verfahren hergestellt werden.

Die Umsetzung von Verbindungen der Formel II mit solchen der Formel IX kann z.B. wie die der Verbindungen der Formel II mit solchen der Formel V erfolgen.

Man kann auch eine Verbindung der Formel IV zuerst in eine andere Verbindung der Formel IV überführen, und erst diese dann in eine Verbindung der Formel I umwandeln. So lässt sich beispielsweise ein Phosphorsäure-niederalkylester-thiazolidinylester der Formel IV durch die Umsetzung mit Säurechloriden von Schwefel- oder Phosphorhaltigen Säuren, wie z.B. Thionylchlorid oder Phosphorpentachlorid, in einem inerten Lösungsmittel, wie einem chlorierten Kohlenwasserstoff, z.B. Chloroform, in einen Phosphorsäure-chlorid-niederalkylester-thiazolidinylester der Formel IV überführen.

Alternativ ist z.B. eine Reaktionsführung möglich, bei der ein Phosphorsäure-diniederalkylester-thiazolidinylester der Formel IV in einen Phosphorsäure-thiazolidinylester der Formel IV übergeführt wird, indem die beiden Niederalkoxygruppen z.B. durch die Umsetzung mit Halogensilanen der Formel $HalSi(R)_3$ mit anschliessender Hydrolyse der als Primärprodukte entstehenden Silylester durch Hydroxygruppen ersetzt werden.

In der Formel $HalSi(R)_3$ bedeutet Hal Chlor, Brom oder Iod und $(R)_3$ steht für eine Kombination von drei gleichen oder verschiedenen Resten der Art Niederalkyl, wie tert.-Butyl, Ethyl oder Methyl, oder substituiertes oder unsubstituiertes Aryl, wie Phenyl. Entsprechende Silane sind z.B. Chlortrimethylsilan, Chlortriethylsilan, tert.-Butylchlordimethylsilan, Chlormethyldiphenylsilan oder die entsprechenden Brom- oder Iod-Verbindungen. Insbesondere die entsprechenden Iod-Verbindungen, aber auch die Brom-Verbindungen, können in situ hergestellt werden, indem z.B. eine der erwähnten Chlor-Verbindungen mit einem Alkali- oder Erdalkalimetalliodid (-bromid), wie Lithiumiodid (-bromid), Natriumiodid (-bromid) oder Magnesiumbromid, umgesetzt wird. Als Lösungsmittel für diese Umsetzungen sind aprotische Lösungsmittel geeignet, wie z.B. Acetonitril, Methylenchlorid oder Tetrachlorkohlenstoff.

Ueberführung von Verbindungen der Formel I ineinander: Erfindungsgemäss erhältliche Verbindungen der Formel I können in an sich bekannter Weise in andere Verbindungen der Formel I übergeführt werden. So kann in Verbindungen der Formel I, in welchen X für Niederalkoxy und insbesondere Methoxy steht, ein solcher Rest in Hydroxy umgewandelt werden, z.B. indem solche Verbindungen mit geeigneten nucleophilen Reagentien behandelt werden, wobei letztere vorzugsweise eine veretherbare Hydroxy- oder insbesondere Mercaptogruppe oder eine substituierbare, inkl. quaternisierbare, Aminogruppe enthalten. Solche Reagentien sind u.a. eine gegebenenfalls substituierte Thiophenolat-Verbindung, wie Thiophenol in Gegenwart einer anorganischen oder organischen Base, wie Triethylamin, ein geeignetes Sulfid, wie Dimethylsulfid in Gegenwart einer Sulfonsäure, wie Methylsulfonsäure, eine geeignete Harnstoff-oder insbesondere Thioharnstoff-Verbindung, wie Thioharnstoff, ferner eine geeignete, vorzugsweise sterisch gehinderte Aminverbindung, wie ein entsprechendes Niederalkylamin, z.B. tert.-Butylamin, ferner Triniederalkylamin, wie Trimethylamin, N-Niederalkyl-morpholin oder -thiomorpholin, z.B. N-Methylmorpholin, oder Pyridin.

Eine weitere Möglichkeit, Verbindungen der Formel I, in denen X für Niederalkoxy und insbesondere Methoxy steht, in Verbindungen der Formel I zu überführen, in denen X Hydroxy bedeutet, bietet die Umsetzung mit Halogensilanen der Formel $HalSi(R)_3$ mit anschliessender Hydrolyse der als Primärprodukte entstehenden Silylester. Sowohl Halogensilane der Formel $HalSi(R)_3$ als auch deren Verwendung sind oben beschrieben.

Darüberhinaus können aus den obengenannten Estern der Formel I freie Säuren der Formel I (X bedeutet Hydroxy) erhalten werden, indem ein Niederalkylester der Formel I (X bedeutet Niederalkoxy), insbesondere ein Methylester, unter Phasentransferbedingungen entalkyliert wird. Als Reagentien hierfür kommen Alkalihalogenide, wie Natriumchlorid oder Natriumbromid, und/oder quartäre Ammoniumsalze, wie z.B. Benzyltriethylammoniumchlorid oder -bromid, in Frage. Das Halogenanion muss äquimolar eingesetzt werden, die Mengen an Ammoniumsalz können sich auf katalytische Mengen beschränken, sofern ein Alkalihalogenid Halogenanionen liefert. Als Lösungsmittel sind aprotische polare, wie Aceton, Acetonitril oder Dimethylformamid, oder aprotische unpolare, wie Benzol oder Toluol, geeignet. Man arbeitet vorzugsweise bei erhöhter Temperatur, z.B. zwischen 30 und 100°C.

Ferner können Verbindungen der Formel I, in denen X Hydroxy ist, in Verbindungen der Formel I übergeführt werden, in denen X für eine Aminogruppe $NR_8R_9$ steht, indem man z.B. die Hydroxygruppe z.B. durch Umsetzung mit Carbonyldiimidazol oder Dicyclohexylcarbodiimid, in eine reaktionsfähige Form bringt, und das Zwischenprodukt durch Behandeln mit einem Amin der Formel $HNR_8R_9$ (VI) in einen Phosphorsäure-diamid-ester der Formel I überführt.

Ferner kann man in Verbindungen der Formel I, worin X für $OR_7$ und $R_7$ für Wasserstoff steht, diesen z.B. durch Behandeln der entsprechenden Verbindung mit einem reaktionsfähigen Ester eines Niederalkanols und einer starken Säure, wie einem entsprechenden Niederalkylhalogenid, z.B. -chlorid, -bromid oder -iodid, oder einem entsprechenden Aren- oder Niederalkansulfonsäure- z.B. p-Toluolsulfonsäure- oder Methansulfonsäure-niederalkylester, durch Niederalkyl ersetzen.

Auch Verbindungen der Formel I, in denen $R_6$ mit $R_7$ oder $R_8$ zusammen eine Niederalkylengruppe bildet, lassen sich aus anderen Verbindungen der Formel I erhalten. So kann z.B. eine Verbindung der Formel I, in der $R_6$ und $R_8$ Wasserstoff sind oder in der $R_6$ und $R_7$ Wasserstoff sind, mit einer Verbindung der Formel

$$\text{Hal—Alk—Hal'} \qquad \text{X},$$

worin Hal und Hal' unabhängig voneinander Halogen, wie z.B. Chlor, Brom oder Iod bedeuten, zu einer Verbindung der Formel I umgesetzt werden, in der $R_6$ mit X und der Atomgruppe P-N einen fünf- bis siebengliedrigen Ring bildet. Ausgangsverbindungen der Formel X sind bekannt oder können auf bekannte Weise hergestellt werden.

Die obigen Reaktionen werden in an sich bekannter Weise, in Abwesenheit, vorteilhafterweise jedoch in Anwesenheit eines geeigneten inerten Lösungsmittels, wie eines gegebenenfalls halogenierten Kohlenwasserstoffs, z.B. Benzol oder Methylenchlorid, Niederalkanols, z.B. Methanol, Dimethylsulfoxid oder Acetonitril oder eines Lösungsmittelgemisches, und üblicherweise unter milden Reaktionsbedingungen, vorzugsweise bei Temperaturen zwischen etwa -10°C und etwa 100°C, insbesondere bei Raumtemperatur oder schwach erhöhten Temperaturen bis etwa 50°C, wenn notwendig, in einem geschlossenen Gefäss und/oder unter einer Inertgas-, z.B. Stickstoffatmosphäre, durchgeführt. Dabei können die Reaktionsprodukte in Form der freien Säuren abgetrennt oder direkt in die Salze, z.B. Alkalimetallsalze, übergeführt werden.

Erfindungsgemäss erhältliche Salze von salzbildenden Verbindungen der Formel I können in an sich bekannter Weise, z.B. durch Behandeln mit einem sauren Reagens, wie einer Säure, in die freien Verbindungen oder durch Umsalzen in andere Salze übergeführt werden.

Salze von zur Salzbildung geeigneten Verbindungen der Formel I, insbesondere pharmazeutisch annehmbare Salze wie z.B. die obgenannten, können in an sich bekannter Weise, z.B. durch Behandeln mit einer geeigneten Base, wie einem Alkalimetallhydroxid, Ammoniak oder einem salzbildenden Amin, hergestellt werden.

Gemische von Isomeren können in an sich bekannter Weise in die reinen Isomere aufgetrennt werden, Racematgemische u.a. mittels physikalischer Auftrennung, z.B. fraktionierter Kristallisation oder Destillation, oder Chromatographie, u.a. Hochdruckflüssigkeitschromatographie, und Racemate u.a. unter Bildung von Salzen mit optisch aktiven Basen und Auftrennung der erhaltenen Salzgemische, z.B. durch fraktionierte Kristallisation.

Die Erfindung betrifft auch diejenigen Ausführungsformen des Verfahrens, bei denen man einen Ausgangsstoff unter den Reaktionsbedingungen bildet, oder bei denen eine Reaktionskomponente gegebenenfalls in Form ihrer Salze vorliegt.

Zweckmässig verwendet man für die Durchführung der erfindungsgemässen Reaktionen solche Ausgangsstoffe, die zu den eingangs besonders erwähnten Gruppen von Endstoffen und besonders zu den speziell beschriebenen oder hervorgehobenen Endstoffen führen.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird. Ferner können Ausgangsstoffe in Form von Derivaten verwendet oder in situ, gegebenenfalls unter den Reaktionsbedingungen, gebildet werden.

Gegenstand der vorliegenden Erfindung ist ebenfalls die Verwendung der neuen Verbindungen als pharmakologisch aktive, insbesondere als carcinostatisch aktive Verbindungen. Die zur Verwendung gelangenden

täglichen Dosen von solchen Verbindungen liegen für Mammalien in Abhängigkeit von der Spezies, dem Alter, dem individuellen Zustand und der Applikationsweise zwischen etwa 2 mg und etwa 250 mg, insbesondere zwischen etwa 5 mg und etwa 100 mg pro kg Körpergewicht, und innerhalb dieses Bereiches bei parenteraler Applikation, z.B. intramuskulärer oder subcutaner Injektion, oder intravenöser Infusion, im allgemeinen niedriger als bei enteraler, d.h. oraler oder rektaler Applikation. Die Verbindungen der Formel I und pharmazeutisch annehmbaren Salze von solchen mit salzbildenden Eigenschaften werden oral oder rektal vorzugsweise in Doseneinheitsformen, wie Tabletten, Dragées oder Kapseln bzw. Suppositorien, und parenteral insbesondere als injizierbare Lösungen, Emulsionen oder Suspensionen oder als Infusionslösungen verabreicht, wobei als Lösungen in erster Linie solche von Salzen in Betracht kommen.

Ebenfalls Gegenstand der Erfindung sind die pharmazeutischen Präparate zur enteralen, z.B. oralen oder rektalen, oder zur parenteralen Verabreichung, welche eine therapeutisch wirksame Menge einer Verbindung der Formel I oder eines pharmazeutisch annehmbaren Salzes einer solchen mit salzbildenden Eigenschaften, gegebenenfalls zusammen mit einem pharmazeutisch verwendbaren Trägerstoff oder Trägerstoffgemisch enthalten, wobei Trägerstoffe anorganisch oder organisch, fest oder flüssig sein können. Entsprechende Dosiseinheitsformen, insbesondere zur oralen Verabreichung, z.B. Dragées, Tabletten oder Kapseln, enthalten vorzugsweise von etwa 50 mg bis etwa 500 mg, insbesondere von etwa 100 mg bis etwa 400 mg einer Verbindung der Formel I oder eines pharmazeutisch annehmbaren Salzes einer zur Salzbildung befähigten entsprechenden Verbindung zusammen mit pharmazeutisch verwendbaren Trägerstoffen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder, wenn erwünscht,

Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol. Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polethylenglykol und/oder Titandioxid enthalten, oder Lacklösungen in geeigneten organischen Lösungsmitteln oder Lösungsmittelgemischen, oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Lactose, Bindemitteln, wie Stärken und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine

Kombination des Wirkstoffs mit einer Grundmasse enthalten; als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat, oder Triglyceride verwendet, oder wässrige Injektionssuspensionen, welche viskositätserhöhende Stoffe, z.B. Natriumcarboxymethylcellulose, Sorbit und/oder Dextran und gegebenenfalls Stabilisatoren enthalten.

Die pharmazeutischen Präparate der vorliegenden Erfindung können in an sich bekannter Weise, z.B. mittels konventioneller Misch-, Granulier-, Dragier-, Lösungs- oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Die nachfolgenden Beispiele illustrieren die oben beschriebene Erfindung, schränken jedoch deren Umfang in keiner Weise ein. Temperaturen werden in Celsiusgraden angegeben.

Beispiel 1: Zu einer Lösung von 33 g (0.10 Mol) 5-Hydroxy-3-methyl-2-[[5-methyl-3-(2-methylallyl)-4-oxo-2-thiazolidinyliden]-hydrazono]-4-thiazolidinon und 43 ml Ethyldiisopropylamin in 250 ml Methylenchlorid lässt man unter Rühren 23.6 g (0.15 Mol) Phoshorsäure-chlorid-dimethylamid-methylester tropfen. Man lässt während 24 Stunden bei 25° reagieren und versetzt dann mit 100 ml Wasser von 0°. Man lässt 5 Minuten kräftig rühren, trennt die organische Phase ab, wäscht sie mit Wasser und engt sie nach dem Trocknen über

Magnesiumsulfat im Wasserstrahlvakuum ein. Als Rückstand verbleibt der Phosphorsäure-dimethylamid-methylester-[3-methyl-2-[[5-methyl-3-(2-methylallyl)-4-oxo-2-thiazolidinyliden]-hydrazono]-4-oxo-5-thiazolidinyl]-ester. Smp. 126-128°, IR ($CH_2Cl_2$): 1735 (s), 1610 (s), 1375 (m), 1320 (m), 1045 (m), 995 (s) $cm^{-1}$ und andere.

Beispiel 2: 45 g (0.10 Mol) Phosphorsäure-dimethylamid-methylester-[3-methyl-2-[[5-methyl-3-(2-methylallyl)-4-oxo-2-thiazolidinyliden]-hydrazono]-4-oxo-5-thiazolidinyl]-ester werden in 100 ml Dioxan unter Reinstickstoff gelöst und unter Rühren mit 22 g tert.-Butylamin (0.30 Mol) versetzt. Man lässt insgesamt 15 Stunden bei 50° rühren, wobei sich nach ca. 12 Stunden das Produkt abzuscheiden beginnt. Anschliessend wird mit 100 ml Diethylether versetzt und das ausgefallene tert.-Butylammoniumsalz des Phosphorsäure-dimethylamid-[3-methyl-2-[[5-methyl-3-(2-methylallyl)-4-oxo-2-thiazolidinyliden]-hydrazono]-4-oxo-5-thiazolidinyl]-esters abgenutscht und mit wenig Diethylether nachgewaschen. Smp. 184° (Zers.), IR (KBr): 1730 (s), 1605 (ss), 1375 (m), 1315 (m), 1220 (s), 1060 (s), 1015 (m), 990 (m) $cm^{-1}$ und andere.

Beispiel 3: 50.8 g (0.10 Mol) des tert.-Butylammoniumsalzes des Phosphorsäure-dimethylamid-[3-methyl-2-[[5-methyl-3-(2-methylallyl)-4-oxo-2-thiazolidinyliden]-hydrazono]-4-oxo-5-thiazolidinyl]-esters werden in 250 ml Eisessig gelöst und unter Rühren innert 15 Minuten mit einer Lösung von 9.90 g (0.103 Mol) Methansulfonsäure in 20 ml Eisessig versetzt, wobei das Produkt auszufallen beginnt. Man lässt während 30 Minuten bei Raumtemperatur rühren, nutscht den Phosphorsäure-dimethylamid-[3-methyl-2-[[5-methyl-3-(2-methylallyl)-4-oxo-2-thiazolidinyliden]-hydrazono]-4-oxo-5-thiazolidinyl]-ester ab und wäscht mit 50 ml Eisessig und 150 ml Diethylether nach.

Beispiel 4: 4.4 g (0.01 Mol) Phosphorsäure-dimethylamid-[3-methyl-2-[[5-methyl-3-(2-methylallyl)-4-oxo-2-thiazolidinyliden]-hydrazono]-4-oxo-5-thiazolidinyl]-ester werden in 50 ml Methanol gelöst und unter Rühren mit einer aus 0.23 g (0.01 Mol) Natrium und 5 ml

Methanol bereiteten Natriummethoxidlösung versetzt. Man versetzt mit Diethylether, wobei sich das Natriumsalz des Phosphorsäure-dimethylamid-[3-methyl-2-[[5-methyl-3-(2-methylallyl)-4-oxo-2-thiazolidinyliden]-hydrazono]-4-oxo-5-thiazolidinyl]-esters abscheidet. Dieses wird abgenutscht, mit wenig Diethylether nachgewaschen und dann 15 Stunden im Hochvakuum bei 50° getrocknet. IR (KBr): 1730 (s), 1615 (s), 1430 (m), 1385 (s), 1325 (m), 1240 (s), 1075 (s), 1020 (m), 990 (m), 925 (m), 840 (m), 750 (m), 735 (m) $cm^{-1}$.

Beispiel 5: Zu einer Suspension von 4.5 g (0.01 Mol) Phosphorsäure-dimethylamid-methylester-[3-methyl-2-[[5-methyl-3-(2-methylallyl)-4-oxo-2-thiazolidinyliden]-hydrazono]-4-oxo-5-thiazolidinyl]-ester in 25 ml 2-Propanol werden 2.2 g (0.02 Mol) Thiophenol gegeben. Man tropft unter Rühren 3.04 ml (0.03 Mol) Triethylamin zu, wobei die Reaktionstemperatur auf 30° ansteigt. Man erwärmt auf 40°, bis die Lösung klar wird, lässt auf Raumtemperatur abkühlen und hält das Reaktionsgemisch 24 Stunden bei dieser Temperatur. Dann kühlt man auf 5° ab und tropft 4 ml einer 2.5-n methanolischen Natriummethylatlösung zu, wobei sich ein gallertartiger Niederschlag bildet. Man verdünnt mit 25 ml Ether und nutscht ab. Das so erhaltene Natriumsalz des Phosphorsäure-dimethylamid-[3-methyl-2-[[5-methyl-3-(2-methylallyl)-4-oxo-2-thiazolidinyliden]-hydrazono]-4-oxo-5-thia-zolidinyl]-esters wird aus Methanol/2-Propanol umkristallisiert. Smp. 201° (Zers.). IR (KBr): 1730 (s), 1615 (s), 1430 (m), 1385 (s), 1325 (m), 1240 (s), 1075 (s), 1020 (m), 990 (m), 925 (m), 840 (m), 750 (m), 735 (m) $cm^{-1}$.

Beispiel 6: Zu 6,6 g (0,02 Mol) 5-Hydroxy-3-methyl-2-[[5-methyl-3-(2-methylallyl)-4-oxo-2-thiazolidinyliden]-hydrazono]-4-thiazolidinon in 30 ml Methylenchlorid gibt man unter Stickstoffatmosphäre und Rühren 4,7 g (0,03 Mol) 2-Chlor-3-methyl-1,3,2-oxazaphospholidin-2-oxid [hergestellt nach A. Takamizawa et al., Chem. Pharm. Bull, 25, 2900 (1977)]. Zu diesem Gemisch wird bei 3° innert 15 Minuten eine Lösung von 3,35 ml (2,43 g; 0,024 Mol) Triethylamin in 10 ml Methylenchlorid zugetropft. Man lässt 90 Minuten im Eisbad und eine Stunde bei Raumtemperatur rühren. Dann versetzt man mit 100 ml

Diethylether und schüttelt im Scheidetrichter zweimal mit je 100 ml Wasser aus. Die organische Phase wird über Magnesiumsulfat getrocknet und dann im Wasserstrahlvakuum eingedampft. Als Rückstand verbleibt das 3-Methyl-2-[3-methyl-2-[[5-methyl-3-(2-methylallyl)-4-oxo-2-thiazolidinyliden]-hydrazono]-4-oxo-5-thiazolidinoxy]-2-oxo-1,3,2-oxazaphospholidin, das zur Reinigung aus Methylenchlorid-Isopropanol umkristallisiert wird. Smp. 158-160°. IR: ($CH_2Cl_2$): 1740, 1615, 1380, 1045, 1005, 940, 845 $cm^{-1}$.

Beispiel 7: In analoger Weise, wie in Beispiel 6 beschrieben, erhält man ausgehend von 7,5 g (0,025 Mol) 3-Allyl-5-hydroxy-2-[[3-methyl-4-oxo-2-thiazolidinyliden]-hydrazono]-4-thiazolidinon, 7,8 g (0,05 Mol) 2-Chlor-3-methyl-1,3,2-oxazaphospholidin-2-oxid und 4,53 ml (0,0325 Mol) Triethylamin in 50 ml Methylenchlorid das 2-[3-Allyl-2-[[3-methyl-4-oxo-2-thiazolidinyliden]-hydrazono]-4-oxo-5-thiazolidinoxy]-3-methyl-2-oxo-1,3,2-oxazaphospholidin vom Smp. 184-185°. IR ($CH_2Cl_2$): 1740, 1650, 1380, 1040, 1005, 940, 845 $cm^{-1}$.

Beispiel 8: In analoger Weise, wie in den vorstehenden Beispielen beschrieben, lassen sich die folgenden erfindungsgemässen Verbindungen herstellen:

Phosphorsäure-di(2-chlorethyl)amid-[3-methyl-2-[[5-methyl-3-(2-methylallyl)-4-oxo-2-thiazolidinyliden]-hydrazono]-4-oxo-5-thiazolidinyl]-ester und seine Salze,

Phosphorsäure-di(2-chlorethyl)amid-methylester- [3-methyl-2-[[5-methyl-3-(2-methylallyl)-4-oxo-2-thiazolidinyliden]-hydrazono]-4-oxo-5-thiazolidinyl]-ester, IR: 1740, 1615, 1380, 1040, 1000 $cm^{-1}$,

Phosphorsäure-bisdimethylamid-[3-methyl-2-[[5-methyl-3-(2-methylallyl)-4-oxo-2-thiazolidinyliden]-hydrazono]-4-oxo-5-thiazolidinyl]-ester, IR: 1740, 1610, 1375, 1045, 1005 $cm^{-1}$,

Phosphorsäure-di(2-chlorethyl)amid-dimethylamid-[3-methyl-2-[[5-methyl-3-(2-methylallyl)-4-oxo-2-thiazolidinyliden]-hydrazono]-4-oxo-5-thiazolidinyl]-ester, IR: 1740, 1615, 1380, 1040, 1005 $cm^{-1}$,

2-[3-(Methyl-2-[[5-methyl-3-(2-methylallyl)-4-oxo-2-thiazolidinyliden]-hydrazono]-4-oxo-5-thiazolidinoxy]-2-oxo-1,3,2-oxazaphospholidin, IR: 1740, 1615, 1380, 1040, 1005, 940, 845 $cm^{-1}$,

2-[3-Methyl-2-[[5-methyl-3-(2-methylallyl)-4-oxo-2-thiazolidinyliden]-hydrazono]-4-oxo-5-thiazolidinoxy]-2-oxo-1,3,2-diazaphospholidin, IR: 1740, 1615, 1375, 1040, 1000 $cm^{-1}$,
1,3-Dimethyl-2-[3-methyl-2-[[5-methyl-3-(2-methylallyl)-4-oxo-2-thiazolidinyliden]-hydrazono]-4-oxo-5-thiazolidinoxy]-2-oxo-1,3,2-diazaphospholidin, IR: 1740, 1615, 1380, 1040, 1005 $cm^{-1}$.

Beispiel 9: Lacktabletten, enthaltend 300 mg Natriumsalz des Phosphorsäure-dimethylamid-[3-methyl-2-[[5-methyl-3-(2-methylallyl)-4-oxo-2-thiazolidinyliden]-hydrazono]-4-oxo-5-thiazolidinyl]-esters können wie folgt hergestellt werden:

Zusammensetzung für 10 000 Tabletten

| | |
|---|---|
| Natriumsalz des Phosphorsäure-dimethylamid-[3-methyl-2-[[5-methyl-3-(2-methylallyl)-4-oxo-2-thiazolidinyliden]-hydrazono]-4-oxo-5-thiazolidinyl]-esters | 3'000.0 g |
| Maisstärke | 630.0 g |
| Kolloidale Kieselsäure | 200.0 g |
| Magnesiumstearat | 20.0 g |
| Stearinsäure | 50.0 g |
| Natriumcarboxymethylstärke | 250.0 g |
| Wasser | q.s. |

Ein Gemisch des Natriumsalzes des Phosphorsäure-dimethylamid-[3-methyl-2-[[5-methyl-3-(2-methylallyl)-4-oxo-2-thiazolidinyliden]-hydrazono]-4-oxo-5-thiazolidinyl]-esters, 50 g Maisstärke und der kolloidalen Kieselsäure wird mit einem Stärkekleister aus 250 g Maisstärke und 2,2 kg entmineralisiertem Wasser zu einer feuchten Masse verarbeitet. Diese wird durch ein Sieb von 3 mm Maschenweite getrieben und bei 45° während 30 Minuten im Wirbelschichttrockner getrocknet. Das trockene Granulat wird durch ein Sieb von 1 mm Maschenweite gedrückt, mit einer zum voraus gesiebten Mischung (1 mm

Sieb) von 330 g Maisstärke, des Magnesiumstearats, der Stearinsäure und der Natriumcarboxymethylstärke gemischt und zu schwach gewölbten Tabletten verpresst.

Die Presslinge werden in einem Dragierkessel von 45 cm Durchmesser mit einer Lösung von 20 g Schellack und 40 g Hydroxypropyl-methylcellulose (niedere Viskosität) in 110 g Methanol und 1350 g Methylenchlorid durch gleichmässiges Aufsprühen während 30 Minuten überzogen; dabei wird durch gleichzeitiges Einblasen von Luft von 60° getrocknet.

Anstelle des obengenannten Wirkstoffes kann auch dieselbe Menge eines anderen Wirkstoffes der vorangehenden Beispiele, wie z.B. der Phosphorsäure-dimethylamid-methylester-[3-methyl-2-[[5-methyl-3-(2-methylallyl)-4-oxo-2-thiazolidinyliden]-hydrazono]-4-oxo-5-thiazolidinyl]-ester, das tert.-Butylammoniumsalz des Phosphorsäure-dimethylamid-[3-methyl-2-[[5-methyl-3-(2-methylallyl)-4-oxo-2-thiazolidinyliden]-hydrazono]-4-oxo-5-thiazolidinyl]-esters oder der Phosphorsäure-dimethylamid-[3-methyl-2-[[5-methyl-3-(2-methylallyl)-4-oxo-2-thiazolidinyliden]-hydrazono]-4-oxo-5-thiazolidinyl]-ester verwendet werden.

Beispiel 10: Je 300 mg Natriumsalz des Phosphorsäure-dimethylamid-[3-methyl-2-[[5-methyl-3-(2-methylallyl)-4-oxo-2-thiazolidinyliden]-hydrazono]-4-oxo-5-thiazolidinyl]-esters vermischt mit Reisstärke, werden in Hartgelatine-Kapseln abgefüllt.

Anstelle des obigen Wirkstoffes kann auch dieselbe Menge eines anderen Wirkstoffes der vorangehenden Beispiele, z.B. des tert.-Butylammoniumsalzes des Phosphorsäure-dimethylamid-[3-methyl-2-[[5-methyl-3-(2-methylallyl)-4-oxo-2-thiazolidinyliden]-hydrazono]-4-oxo-5-thiazolidinyl]-esters, verwendet werden.

Beispiel 11: Je 5 ml einer sterilen, 4%igen wässrigen Lösung vom Natriumsalz des Phosphorsäure-dimethylamid-[3-methyl-2-[[5-methyl-3-(2-methylallyl)-4-oxo-2-thiazolidinyliden]-hydrazono]-4-oxo-5-thiazolidinyl]-esters, entsprechend 200 mg Wirkstoff, werden in Ampullen eingefüllt und diese verschlossen und geprüft.

Anstelle des obigen Wirkstoffes kann auch dieselbe Menge eines anderen Wirkstoffes der vorangehenden Beispiele, z.B. des tert.-Butylammoniumsalzes des Phosphorsäure-dimethylamid-[3-methyl-2-[[5-methyl-3-(2-methylallyl)-4-oxo-2-thiazolidinyliden]-hydrazono]-4-oxo-5-thiazolidinyl]-esters, verwendet werden.

Patentansprüche (für alle Vertragsstaaten ausser Oesterreich)

1. Verbindungen der Formel

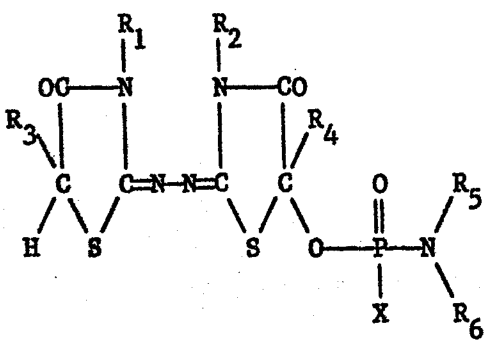

I,

in welcher eines der Symbole $R_1$ und $R_2$ einen in der 2,3-Stellung ungesättigten Alkylrest mit 3 oder 4 Kohlenstoffatomen und das andere einen solchen Rest oder Niederalkyl bedeuten, $R_3$ und $R_4$ unabhängig voneinander Wasserstoff oder Methyl darstellen, $R_5$ und $R_6$ unabhängig voneinander Wasserstoff, Niederalkyl, halogeniertes Niederalkyl oder Niederalkenyl oder zusammen gegebenenfalls durch Sauerstoff, Schwefel oder gegebenenfalls substituierten Stickstoff unterbrochenes Niederalkylen bedeuten, X $OR_7$ oder $NR_8R_9$ bedeutet, wobei $R_7$ für Wasserstoff oder Niederalkyl steht, und $R_8$ und $R_9$ unabhängig voneinander Wasserstoff, Niederalkyl, halogeniertes Niederalkyl oder Niederalkenyl oder zusammen gegebenenfalls durch Sauerstoff, Schwefel oder gegebenenfalls substituierten Stickstoff unterbrochenes Niederalkylen bedeuten, oder $R_6$ zusammen mit $R_7$ oder mit $R_8$ eine Niederalkylengruppe bedeutet, Salze von Verbindungen der allgemeinen Formel I, in denen $R_7$ Wasserstoff bedeutet, die reinen Isomere von Verbindungen der Formel I und Mischungen dieser Isomere.

2. Verbindungen der Formel I gemäss Anspruch 1, in denen einer der Reste $R_1$ und $R_2$ in 2,3-Stellung ungesättigtes Alkyl mit 3 oder 4 Kohlenstoffatomen, z.B. entsprechendes Niederalkenyl oder entsprechendes Niederalkinyl, und der andere ebenfalls eine dieser Gruppen

oder Niederalkyl mit bis zu 4 Kohlenstoffatomen bedeuten, $R_3$ und $R_4$ unabhängig voneinander Wasserstoff oder Methyl darstellen, $R_5$ und $R_6$ unabhängig voneinander Wasserstoff, Niederalkyl, ein- oder zweifach halogeniertes Niederalkyl oder Niederalkenyl oder zusammen gegebenenfalls durch Sauerstoff, Schwefel oder gegebenenfalls niederalkylierten Stickstoff unterbrochenes Niederalkylen bedeuten, X $OR_7$ oder $NR_8R_9$ bedeutet, worin $R_7$ für Wasserstoff oder Niederalkyl steht, und $R_8$ und $R_9$ unabhängig voneinander Wasserstoff, Niederalkyl, ein- oder zweifach halogeniertes Niederalkyl oder Niederalkenyl oder zusammen gegebenenfalls durch Sauerstoff, Schwefel oder gegebenenfalls niederalkylierten Stickstoff unterbrochenes Niederalkylen bedeuten, oder $R_6$ zusammen mit $R_7$ oder mit $R_8$ Niederalkylen mit 2 bis 4 Kohlenstoffatomen bedeutet, und Salze von solchen Verbindungen der Formel I, worin $R_7$ Wasserstoff ist.

3. Verbindungen der Formel I gemäss Anspruch 1, worin einer der Reste $R_1$ und $R_2$ Allyl, 1-Methylallyl, 2-Methylallyl oder 2-Propinyl und der andere ebenalls eine dieser Gruppen oder Methyl ist, $R_3$ und $R_4$ unabhängig voneinander für Wasserstoff oder Methyl stehen, $R_5$ und $R_6$ unabhängig voneinander Wasserstoff, Niederalkyl oder durch Chlor mono- oder disubstituiertes Niederalkyl oder zusammen gegebenenfalls durch Sauerstoff, Schwefel oder gegebenenfalls methylierten Stickstoff unterbrochenes Niederalkylen bedeuten, X $OR_7$ oder $NR_8R_9$ bedeutet, worin $R_7$ Wasserstoff oder Niederalkyl ist, und $R_8$ und $R_9$ unabhängig voneinander Wasserstoff, Niederalkyl oder durch Chlor mono- oder disubstituiertes Niederalkyl oder zusammen gegebenenfalls durch Sauerstoff, Schwefel oder gegebenenfalls methylierten Stickstoff unterbrochenes Niederalkylen bedeuten, oder $R_6$ zusammen mit $R_7$ oder mit $R_8$ Niederalkylen mit 2 bis 4 Kohlenstoffatomen bedeutet, und Salze von solchen Verbindungen der Formel I, worin $R_7$ Wasserstoff ist.

4. Verbindungen der Formel I gemäss Anspruch 1, worin einer der Reste $R_1$ und $R_2$ Allyl oder 2-Methylallyl und der andere Methyl ist, $R_3$ Wasserstoff oder Methyl bedeutet, $R_4$ Wasserstoff ist, $R_5$ und $R_6$ unabhängig voneinander Wasserstoff, Methyl oder 2-Chlorethyl

bedeuten, X $OR_7$ oder $NR_8R_9$ ist, worin $R_7$, $R_8$ und $R_9$ Wasserstoff oder Methyl sind, oder $R_6$ zusammen mit $R_7$ Ethylen bedeutet, das zusammen mit der Atomgruppe O-P-N einen fünfgliedrigen Ring bildet, oder $R_6$ zusammen mit $R_8$ Ethylen bedeutet, das zusammen mit der Atomgruppe N-P-N einen fünfgliedrigen Ring bildet, und Salze von solchen Verbindungen der Formel I, in denen $R_7$ Wasserstoff ist.

5. Verbindungen der Formel I gemäss Anspruch 1, worin einer der Reste $R_1$ und $R_2$ Allyl oder 2-Methylallyl und der andere Methyl ist, $R_3$ für Wasserstoff oder Methyl steht, $R_4$ Wasserstoff ist, $R_5$ und $R_6$ Methyl sind, X $OR_7$ ist, worin $R_7$ Wasserstoff oder Methyl ist, oder $R_6$ zusammen mit $R_7$ Ethylen bedeutet, das zusammen mit der Atomgruppe -O-P-N- einen fünfgliedrigen Ring bildet, und Salze von solchen Verbindungen der Formel I, worin $R_7$ Wasserstoff ist.

6. Pharmazeutisch annehmbare Salze von Verbindungen der Formel I gemäss Anspruch 1.

7. Die reinen optischen Antipoden von Verbindungen der Formel I gemäss Anspruch 1 und Mischungen dieser optischen Antipoden.

8. Phosphorsäure-dimethylamid-methylester-[3-methyl-2-[[5-methyl-3-(2-methylallyl)-4-oxo-2-thiazolidinyliden]-hydrazono]-4-oxo-5-thiazolidinyl]ester gemäss Anspruch 1.

9. Phosphorsäure-dimethylamid-[3-methyl-2-[[5-methyl-3-(2-methylallyl)-4-oxo-2-thiazolidinyliden]-hydrazono]-4-oxo-5-thiazolidinyl]-ester und pharmazeutisch annehmbare Salze davon gemäss Anspruch 1.

10. 3-Methyl-2-[3-methyl-2-[[5-methyl-3-(2-methylallyl)-4-oxo-2-thiazolidinyliden]-hydrazono]-4-oxo-5-thiazolidinoxy]-2-oxo-1,3,2-oxazaphospholidin gemäss Anspruch 1.

11. 2-[3-Allyl-2-[[3-methyl-4-oxo-2-thiazolidinyliden]-hydrazono]-4-oxo-5-thiazolidinoxy]-3-methyl-2-oxo-1,3,2-oxazaphospholidin gemäss Anspruch 1.

12. Verbindungen der Formel I gemäss Anspruch 1 und pharmazeutisch annehmbare Salze davon als tumorhemmende Mittel.

13. Verbindungen der Formel I gemäss Anspruch 1 und pharmazeutisch annehmbare Salze davon zur Anwendung in einem Verfahren zur therapeutischen Behandlung des menschlichen oder tierischen Körpers.

14. Pharmazeutische Präparate enthaltend Verbindungen der Formel I gemäss Anspruch 1 oder pharmazeutisch annehmbare Salze von solchen Verbindungen.

15. Verwendung von Verbindungen der Formel I gemäss Anspruch 1 oder pharmazeutisch annehmbaren Salzen von solchen Verbindungen zur Herstellung von pharmazeutischen Präparaten.

16. Verfahren zur Herstellung von Verbindungen der Formel

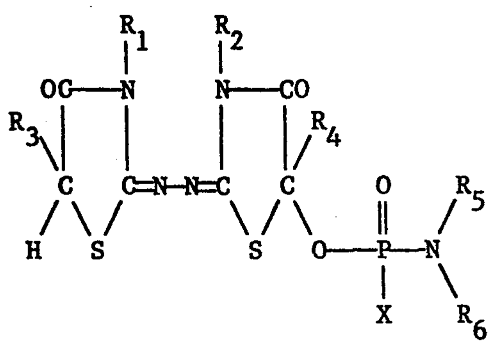

I,

in welcher eines der Symbole $R_1$ und $R_2$ einen in der 2,3-Stellung ungesättigten Alkylrest mit 3 oder 4 Kohlenstoffatomen und das andere einen solchen Rest oder Niederalkyl bedeuten, $R_3$ und $R_4$ unabhängig voneinander Wasserstoff oder Methyl darstellen, $R_5$ und $R_6$ unabhängig voneinander Wasserstoff, Niederalkyl, halogeniertes Niederalkyl oder Niederalkenyl oder zusammen gegebenenfalls durch Sauerstoff, Schwefel oder gegebenenfalls substituierten Stickstoff

unterbrochenes Niederalkylen bedeuten, X $OR_7$ oder $NR_8R_9$ bedeutet, worin $R_7$ für Wasserstoff oder Niederalkyl steht, und $R_8$ und $R_9$ unabhängig voneinander Wasserstoff, Niederalkyl, halogeniertes Niederalkyl oder Niederalkenyl oder zusammen gegebenenfalls durch Sauerstoff, Schwefel oder gegebenenfalls substituierten Stickstoff unterbrochenes Niederalkylen bedeuten, oder $R_6$ zusammen mit $R_7$ oder mit $R_8$ eine Niederalkylengruppe bedeutet, Salzen von Verbindungen der Formel I, in denen $R_7$ Wasserstoff bedeutet, reinen Isomeren von Verbindungen der Formel I und Mischungen dieser Isomeren, dadurch gekennzeichnet, dass man

a) eine Verbindung der Formel

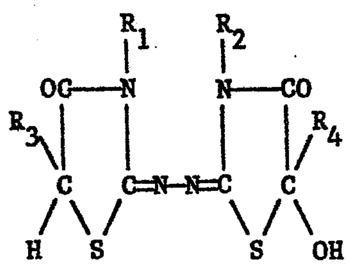

II,

mit einer den Phosphorsäurerest der Formel

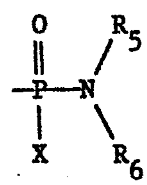

III

einführenden Verbindung umsetzt, oder

b) in einer Verbindung der Formel

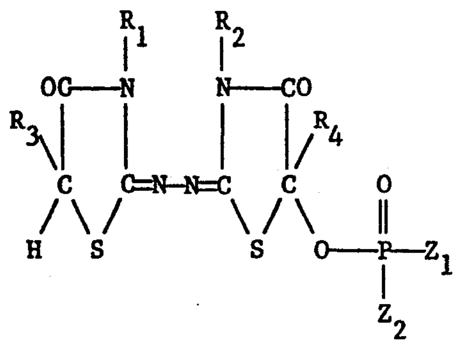

IV,

worin $Z_1$ einen durch die Gruppe $NR_5R_6$ ersetzbaren Rest oder $NR_5R_6$ und $Z_2$ einen durch die Gruppe X ersetzbaren Rest oder X bedeuten, mit der Massgabe, dass mindestens eine der Gruppen $Z_1$ und $Z_2$ von der Gruppe $NR_5R_6$ bzw. X verschieden ist, den Rest $Z_1$ durch die Gruppe $NR_5R_6$ und/oder den Rest $Z_2$ durch die Gruppe X ersetzt, und gewünschtenfalls eine erhältliche Verbindung der allgemeinen Formel I in eine andere Verbindung der allgemeinen Formel I überführt, und/oder gewünschtenfalls ein verfahrensgemäss erhältliches Salz in die freie Verbindung oder in ein anderes Salz überführt und/oder eine verfahrensgemäss erhältliche Verbindung der Formel I, in der $R_7$ Wasserstoff bedeutet, in ein Salz derselben überführt, und/oder, wenn erwünscht, ein verfahrensgemäss erhältliches Isomerengemisch in die einzelnen Isomeren auftrennt.

17. Die nach dem Verfahren gemäss Anspruch 16 erhältlichen Verbindungen.

Patentansprüche für den Vertragsstaat Oesterreich

1. Verfahren zur Herstellung von Verbindungen der Formel

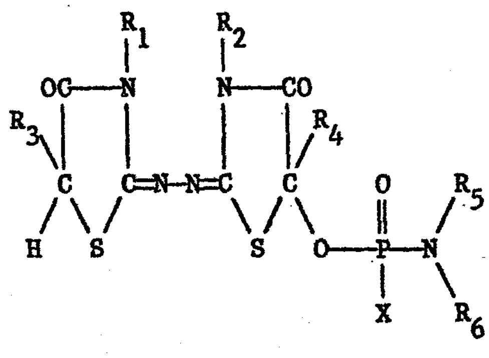

I,

in welcher eines der Symbole $R_1$ und $R_2$ einen in der 2,3-Stellung ungesättigten Alkylrest mit 3 oder 4 Kohlenstoffatomen und das andere einen solchen Rest oder Niederalkyl bedeuten, $R_3$ und $R_4$ unabhängig voneinander Wasserstoff oder Methyl darstellen, $R_5$ und $R_6$ unabhängig voneinander Wasserstoff, Niederalkyl, halogeniertes Niederalkyl oder Niederalkenyl oder zusammen gegebenenfalls durch Sauerstoff, Schwefel oder gegebenenfalls substituierten Stickstoff unterbrochenes Niederalkylen bedeuten, X $OR_7$ oder $NR_8R_9$ bedeutet, worin $R_7$ für Wasserstoff oder Niederalkyl steht, und $R_8$ und $R_9$ unabhängig voneinander Wasserstoff, Niederalkyl, halogeniertes Niederalkyl oder Niederalkenyl oder zusammen gegebenenfalls durch Sauerstoff, Schwefel oder gegebenenfalls substituierten Stickstoff unterbrochenes Niederalkylen bedeuten, oder $R_6$ zusammen mit $R_7$ oder mit $R_8$ eine Niederalkylengruppe bedeutet, Salzen von Verbindungen der Formel I, in denen $R_7$ Wasserstoff bedeutet, reinen Isomeren von Verbindungen der Formel I und Mischungen dieser Isomeren, dadurch gekennzeichnet, dass man

a) eine Verbindung der Formel

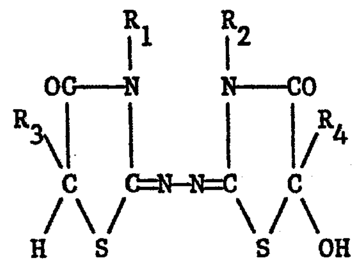

II,

mit einer den Phosphorsäurerest der Formel

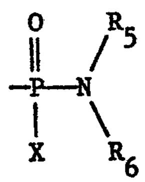

III

einführenden Verbindung umsetzt, oder

b) in einer Verbindung der Formel

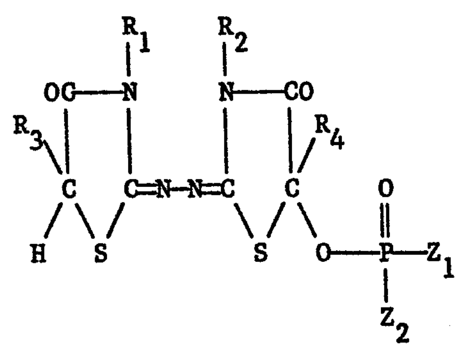

IV,

worin $Z_1$ einen durch die Gruppe $NR_5R_6$ ersetzbaren Rest oder $NR_5R_6$ und $Z_2$ einen durch die Gruppe X ersetzbaren Rest oder X bedeuten, mit der Massgabe, dass mindestens eine der Gruppen $Z_1$ und $Z_2$ von der Gruppe $NR_5R_6$ bzw. X verschieden ist, den Rest $Z_1$ durch die Gruppe $NR_5R_6$ und/oder den Rest $Z_2$ durch die Gruppe X ersetzt, und gewünschtenfalls eine erhältliche Verbindung der allgemeinen Formel I in eine andere Verbindung der allgemeinen Formel I überführt, und/oder gewünschtenfalls ein verfahrensgemäss erhältliches Salz in die freie Verbindung oder in ein anderes Salz überführt und/oder eine verfahrensgemäss erhältliche Verbindung der Formel I, in der $R_7$ Wasser-

stoff bedeutet, in ein Salz derselben überführt, und/oder, wenn erwünscht, ein verfahrensgemäss erhältliches Isomerengemisch in die einzelnen Isomeren auftrennt.

2. Verfahren zur Herstellung von Verbindungen der Formel I gemäss Anspruch 1, worin einer der Reste $R_1$ oder $R_2$ in 2,3-Stellung ungesättigtes Alkyl mit 3 oder 4 Kohlenstoffatomen, z.B. entsprechendes Niederalkenyl oder entsprechendes Niederalkinyl, und der andere ebenfalls eine dieser Gruppen oder Niederalkyl mit bis zu 4 Kohlenstoffatomen bedeuten, $R_3$ und $R_4$ unabhängig voneinander Wasserstoff oder Methyl darstellen, $R_5$ und $R_6$ unabhängig voneinander Wasserstoff, Niederalkyl, ein- oder zweifach halogeniertes Niederalkyl oder Niederalkenyl oder zusammen gegebenenfalls durch Sauerstoff, Schwefel oder gegebenenfalls niederalkylierten Stickstoff unterbrochenes Niederalkylen bedeuten, X $OR_7$ oder $NR_8R_9$ bedeutet, worin $R_7$ für Wasserstoff oder Niederalkyl steht, und $R_8$ und $R_9$ unabhängig voneinander Wasserstoff, Niederalkyl, ein- oder zweifach halogeniertes Niederalkyl oder Niederalkenyl oder zusammen gegebenenfalls durch Sauerstoff, Schwefel oder gegebenenfalls niederalkylierten Stickstoff unterbrochenes Niederalkylen bedeuten, oder $R_6$ zusammen mit $R_7$ oder mit $R_8$ Niederalkylen mit 2 bis 4 Kohlenstoffatomen bedeutet, und Salze von solchen Verbindungen der Formel I, worin $R_7$ Wasserstoff ist.

3. Verfahren zur Herstellung von Verbindungen der Formel I gemäss Anspruch 1, worin einer der Reste $R_1$ und $R_2$ Allyl, 1-Methylallyl, 2-Methylallyl oder 2-Propinyl und der andere ebenfalls eine dieser Gruppen oder Methyl ist, $R_3$ und $R_4$ unabhängig voneinander für Wasserstoff oder Methyl stehen, $R_5$ und $R_6$ unabhängig voneinander Wasserstoff, Niederalkyl oder durch Chlor mono- oder disubstituiertes Niederalkyl oder zusammen gegebenenfalls durch Sauerstoff, Schwefel oder gegebenenfalls methylierten Stickstoff unterbrochenes Niederalkylen bedeuten, X $OR_7$ oder $NR_8R_9$ bedeutet, worin $R_7$ Wasserstoff oder Niederalkyl ist, und $R_8$ und $R_9$ unabhängig voneinander Wasserstoff, Niederalkyl oder durch Chlor mono- oder disubstituiertes Niederalkyl oder zusammen gegebenenfalls durch Sauerstoff,

Schwefel oder gegebenenfalls methylierten Stickstoff unterbrochenes Niederalkylen bedeuten, oder $R_6$ zusammen mit $R_7$ oder mit $R_8$ Niederalkylen mit 2 bis 4 Kohlenstoffatomen bedeutet, und Salze von solchen Verbindungen der Formel I, worin $R_7$ Wasserstoff ist.

4. Verfahren zur Herstellung von Verbindungen der Formel I gemäss Anspruch 1, in denen einer der Reste $R_1$ und $R_2$ Allyl oder 2-Methylallyl und der andere Methyl ist, $R_3$ Wasserstoff oder Methyl bedeutet, $R_4$ Wasserstoff ist, $R_5$ und $R_6$ unabhängig voneinander Wasserstoff, Methyl oder 2-Chlorethyl bedeuten, X $OR_7$ oder $NR_8R_9$ ist, worin $R_7$, $R_8$ und $R_9$ Wasserstoff oder Methyl sind, oder $R_6$ zusammen mit $R_7$ Ethylen bedeutet, das zusammen mit der Atomgruppe O-P-N einen fünfgliedrigen Ring bildet, oder $R_6$ zusammen mit $R_8$ Ethylen bedeutet, das zusammen mit der Atomgruppe N-P-N einen fünfgliedrigen Ring bildet, und Salze von solchen Verbindungen der Formel I, in denen $R_7$ Wasserstoff ist.

5. Verfahren zur Herstellung von Verbindungen der Formel I gemäss Anspruch 1, worin einer der Reste $R_1$ und $R_2$ Allyl oder 2-Methylallyl und der andere Methyl ist, $R_3$ für Wasserstoff oder Methyl steht, $R_4$ Wasserstoff ist, $R_5$ und $R_6$ Methyl sind, X $OR_7$ ist, worin $R_7$ Wasserstoff oder Methyl ist, oder $R_6$ zusammen mit $R_7$ Ethylen bedeutet, das zusammen mit der Atomgruppe -O-P-N- einen fünfgliedrigen Ring bildet, und Salze von solchen Verbindungen der Formel I, worin $R_7$ Wasserstoff ist.

6. Verfahren zur Herstellung von pharmazeutisch annehmbaren Salzen von Verbindungen der Formel I gemäss Anspruch 1.

7. Verfahren zur Herstellung der reinen optischen Antipoden von Verbindungen der Formel I gemäss Anspruch 1.

8. Verfahren zur Herstellung von Phosphorsäure-dimethylamid-methylester-[3-methyl-2-[[5-methyl-3-(2-m ethylallyl)-4-oxo-2-thiazolidinyliden]-hydrazono]-4-oxo-5-thiazolidinyl]-ester gemäss Anspruch 1.

9. Verfahren zur Herstellung von Phosphorsäure-dimethylamid-[3-methyl-2-[[5-methyl-3-(2-methylallyl)-4-oxo-2-thiazolidinyliden]-hydrazono]-4-oxo-5-thiazolidinyl]-ester und pharmazeutisch annehmbaren Salzen davon gemäss Anspruch 1.

10. Verfahren zur Herstellung von 3-Methyl-2-[3-methyl-2-[[5-methyl-3-(2-methylallyl)-4-oxo-2-thiazolidinyliden]-hydrazono]-4-oxo-2-thiazolidinoxy]-2-oxo-1,3,2-oxazaphospholidin gemäss Anspruch 1.

11. Verfahren zur Herstellung von 2-[3-Allyl-2-[[3-methyl-4-oxo-2-thiazolidinyliden]-hydrazono]-4-oxo-5-thiazolidinoxy]-3-methyl-2-oxo-1,3,2-oxazaphospholidin gemäss Anspruch 1.

12. Verfahren zur Herstellung von pharmazeutischen Präparaten, dadurch gekennzeichnet, dass man eine gemäss Anspruch 1 herstellbare Verbindung oder ein pharmazeutisch annehmbares Salz einer solchen Verbindung mit einem pharmazeutischen Trägermaterial verarbeitet.

FO 7.4/UL/gs*/jt*